# EUROPEAN PATENT SPECIFICATION

(11) **EP 3 435 923 B1**
(45) Date of publication and mention of the grant of the patent: **28.04.2021**
(21) Application number: 16721937.7
(22) Date of filing: 30.03.2016
(51) Int. Cl.: A61F 2/44, A61F 2/46

(54) **ADJUSTABLE SPINAL CAGE**
EINSTELLBARER WIRBELKÄFIG
CAGE VERTÉBRALE RÉGLABLE

(43) Date of publication of application: 06.02.2019
(73) Proprietor: Apifix Ltd., 2161401 Carmiel (IL)
(72) Inventor: ARNIN, Uri, 3603117 Kiryat Tivon (IL)
(74) Representative: Dentons UK and Middle East LLP
(86) International application number: PCT/IB2016/051800
(87) International publication number: WO 2017/168208

(56) References cited:
- WO-A1-98/14142
- WO-A1-98/48739
- WO-A1-2014/152337
- WO-A2-2014/186384
- US-A- 4 759 769
- US-A1- 2016 030 190

## Description

### FIELD OF THE INVENTION

The present invention relates generally to spinal implants and prostheses, and particularly to an expandable and rotatable spinal cage, for example, with a slidable hinge.

### BACKGROUND OF THE INVENTION

Spine degeneration of different types is affecting significant portion of the population. Current surgical treatment involves many times the use of an intervertebral cage that is placed between two adjacent vertebrae.

As the anatomy is different from case to case, and as inserting a small implant is always easier than placing a bigger one, a cage that can be inserted between the vertebrae in a small configuration and adjusted in situ to fit the anatomy is a clear need.

US 2016/030190 A1 describes an expandable assembly for insertion into an intervertebral space.

### SUMMARY OF THE INVENTION

The present invention seeks to provide an improved expandable spinal cage, which, for example, can control the lordosis angle between two vertebrae after being installed in a contracted configuration.

According to the present invention, the expandable cage has a slidably supported hinge, as is described more in detail hereinbelow.

There is thus provided in accordance with present invention a spinal cage including a first support plate pivotally connected to a second support plate by a hinge, a plate mover actuated by an actuator and located between the first and second support plates, the plate mover being arranged to slide against an inclined surface on the first support plate, and a hinge journaled in an elongate aperture formed in a hinge housing protruding from first support plate, the hinge being free to translate and rotate in the elongate aperture, and one or more support members biased towards the hinge and/or the first support plate.

In accordance with an embodiment of the present invention the one or more support members may be biased by a biasing device.

In accordance with an embodiment of the present invention the inclined surface has different slopes at different portions thereof.

In accordance with an embodiment of the present invention the plate mover has different slopes at different portions thereof.

In accordance with an embodiment of the present invention one or more keels extend from the first support plate and/or the second support plate.

In accordance with an embodiment of the present invention inner ramps are provided on which the first support plate moves.

### BRIEF DESCRIPTION OF THE DRAWINGS

The present invention will be understood and appreciated more fully from the following detailed description taken in conjunction with the drawings in which:
Fig. 1 is a simplified pictorial illustration of a spinal cage, in an initial, contracted configuration, constructed and operative in accordance with a non-limiting embodiment of the invention;
Figs. 2 and 3 are simplified pictorial and end-view illustrations, respectively, of the cage of Fig. 1 in an expanded configuration, in which the cage has been lifted and tilted to a new height and tilt angle (e.g., for achieving a desired height and lordosis angle);
Fig. 4 is a simplified pictorial illustration of a spinal cage, in an initial, contracted configuration, constructed and operative in accordance with another non-limiting embodiment of the invention;
Figs. 5, 6 and 7 are simplified end-view illustrations of the cage of Fig. 4 in respective contracted, expanded height and expanded height plus tilt configurations;
Figs. 8 and 9 are simplified pictorial and side-view illustrations, respectively, inner ramps in the cage, which determine the way in which the plates tilt and move with respect to each other; and
Fig. 10 is a simplified pictorial illustration of an applicator tool and an adjustment tool for installing the spinal cage, constructed and operative in accordance with a non-limiting embodiment of the invention.

### DETAILED DESCRIPTION OF EMBODIMENTS

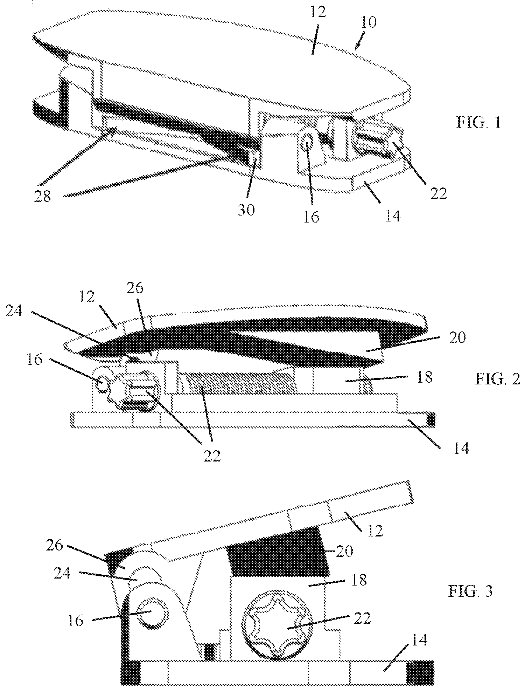

Reference is now made to Figs. 1-3, which illustrate a spinal cage 10, constructed and operative in accordance with a non-limiting embodiment of the invention.

Spinal cage 10 includes a first support plate 12 and a second support plate 14, which may be (but not necessarily) initially parallel to each other. First support plate 12 is pivotally connected to second support plate 14 by a hinge 16. A plate mover 18 is located between first and second support plates 12 and 14. Plate mover 18 may be a wedge with an inclined surface or other suitable pushing structure that may be flat or curved (any geometrical shape). Plate mover 18 is arranged to slide against an inclined surface 20 (having any geometrical shape) on (e.g., the underside of) first support plate 12. As will be described below, the action of plate mover 18 sliding against inclined surface 20 causes first support plate 12 to be lifted and/or tilted with respect to second support plate 14.

Inclined surface 20 (as well as plate mover 18) can have different slopes (in terms of depth, angle, length, shape, size, etc.) at different portions along its length or width.

Plate mover 18 may be actuated by an actuator 22, such as a linear actuator (e.g., a control screw threaded through a boss protruding from second support plate 14). Actuator 22 may be manually operated (e.g., by turning an appropriate screwdriver that mates with the head of the control screw) or automatically operated (e.g., actuator 22 may be a motor, locally or remotely operated, which turns the control screw, such as with feedback from sensors in a control loop).

Hinge 16 is journaled in an elongate aperture 24 (e.g., oval or elliptical aperture) formed in a hinge housing 26 protruding from first support plate 12. Hinge 16 is free to translate and rotate in elongate aperture 24.

In accordance with a non-limiting embodiment of the invention, one or more support members 28 may be biased towards hinge 16 and/or first support plate 12. The support members 28 may be made of a flexible material or may be urged by a biasing device 30 (e.g., a leaf spring or coil spring) Initially, when the spinal cage 10 is in its contracted configuration, the support members 28 simply abut against hinge 16 and/or first support plate 12. After first support plate 12 is lifted and/or tilted with respect to second support plate 14, a gap is formed between hinge 16 and second support plate 14, and/or between first support plate 12 and second support plate 14, and the support members 28 are urged by the biasing force into this gap. In this manner, the support members 28 support and maintain first support plate 12 in its expanded configuration (lifted and/or tilted). The one or more support members 28 may move in translation and/or rotation (e.g., about a pivot).

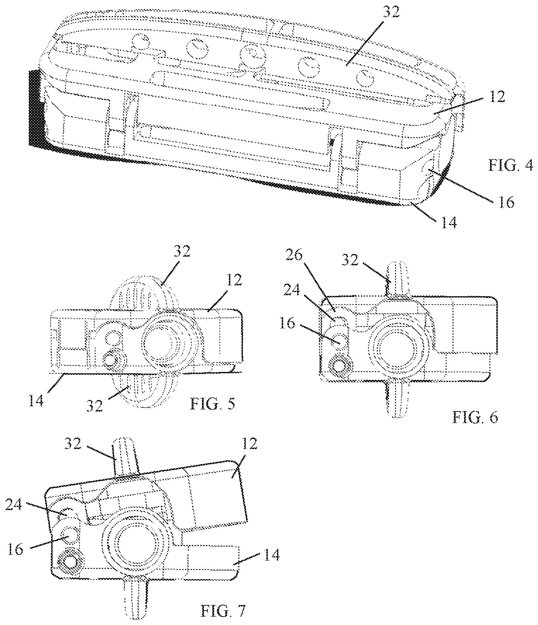

Reference is now made to Figs. 4-7, which illustrate spinal cage 10 with one or more keels 32 extending (e.g., at right angles) from first support plate 12 and/or second support plate 14. The keels 32 may be used to ensure proper installation orientation of the cage in the spinal structure. Figs. 5-7 illustrate cage 10 respectively in contracted, expanded height and expanded height plus tilt configurations.

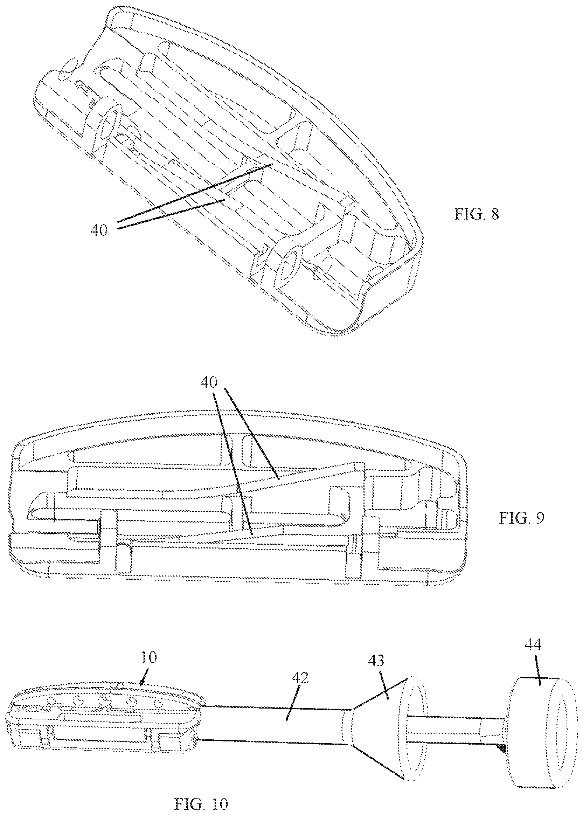

Reference is now made to Figs. 8 and 9, which illustrate one or more inner ramps 40 in the cage 10 on which first support plate 12 moves, which determine the way in which the plates 12 and 14 tilt and move with respect to each other. Ramps 40 serve as the inclined surface 20 or may be in addition to another inclined surface. As seen in the illustrated embodiment, the inclined portion of one of the ramps may have a different length, height, shape or angle than another of the ramps. The hinge and first support plate may slide upwards on one of the ramps until the hinge reaches its highest position in the elongate aperture. Afterwards, sliding continues on the other ramp or ramps to cause tilting of the first support plate. The ramps thus help achieve the desired lordosis angle.

Reference is now made to Fig. 10 is a simplified pictorial illustration of an applicator tool 42 and an adjustment tool 44 for installing the spinal cage, constructed and operative in accordance with a non-limiting embodiment of the invention.

The applicator tool 42 may be a tube with a proximal funnel 43. The adjustment tool 44 may be a screwdriver for turning the actuator, for example. Cage 10 may be attached to the distal portion of applicator tool 42 (such as by a male-female connection, threaded connection, or any other suitable connection) Applicator tool 42 grasps the cage 10 during insertion in the disc space or other structure. The adjustment tool 44 may be inserted through the tube of the applicator tool 42 to reach the actuator. After cage 10 is expanded in situ, the adjustment tool 44 is removed. After lifting or tilting the first support plate, bone graft, collagen or other materials may be added into the spinal cage, such as below the first support plate.

## Claims

1. A spinal cage (10) comprising:
a first support plate (12) pivotally connected to a second support plate (14) by a hinge (16); and
a plate mover (18) actuated by an actuator (22) and located between said first and second support plates (12, 14), said plate mover (18) being arranged to slide against an inclined surface (20) on said first support plate (12);
wherein said hinge (16) is journaled in an elongate aperture (24) formed in a hinge housing (26) protruding from said first support plate (12), said hinge (16) being free to translate and rotate in said elongate aperture (24),
**characterized in that** said spinal cage (10) comprises one or more support members (28) biased towards said hinge (16) and/or said first support plate (12).

2. The spinal cage (10) according to claim 1, wherein said one or more support members (28) are made of a flexible material.

3. The spinal cage (10) according to claim 1, wherein when said spinal cage (10) is in a contracted configuration, said one or more support members (28) abut against said hinge (16) and/or said first support plate (12).

4. The spinal cage (10) according to claim 1, wherein said one or more support members (28) move in translation and/or rotation.

5. The spinal cage (10) according to claim 1, wherein when said first support plate (12) is lifted and/or tilted with respect to said second support plate (14), a gap is formed between said hinge (16) and said second support plate (14), and/or between said first support plate (12) and said second support plate (14), and said one or more support members (28) are urged by a biasing force to move into said gap.

6. The spinal cage (10) according to claim 1 or claim 5, wherein said one or more support members (28) are biased by a biasing device (30).

7. The spinal cage (10) according to claim 6, wherein said biasing device (30) comprises a spring.

8. The spinal cage (10) according to claim 1 or claim 5, wherein said inclined surface (20) has different slopes at different portions thereof.

9. The spinal cage (10) according to claim 1 or claim 5, wherein said plate mover (18) has different slopes at different portions thereof.

10. The spinal cage (10) according to claim 1 or claim 5, further comprising one or more keels (32) extending from said first support plate (12) and/or said second support plate (14).

11. The spinal cage (10) according to claim 1 or claim 5, further comprising inner ramps (40) on which said first support plate (12) moves.

12. The spinal cage (10) according to claim 1 or claim 5, further comprising an applicator tool (42) with a proximal funnel (43) and an adjustment tool (44) for passing through said applicator tool (42).

13. The spinal cage (10) according to claim 5, wherein said one or more support members (28) are made of a flexible material.

14. The spinal cage (10) according to claim 5, wherein when said spinal cage (10) is in a contracted configuration, said one or more support members (28) abut against said hinge (16) and/or said first support plate (12).

15. The spinal cage (10) according to claim 5, wherein said one or more support members (28) move in translation and/or rotation.

## Patentansprüche

1. Wirbelsäulen-Cage (10), das Folgendes umfasst:
eine erste Stützplatte (12), die durch ein Scharnier (16) mit einer zweiten Stützplatte (14) schwenkbar verbunden ist; und
einen Plattenbeweger (18), der durch ein Betätigungselement (22) betätigt wird und sich zwischen der ersten und der zweiten Stützplatten (12, 14) befindet, wobei der Plattenbeweger (18) angeordnet ist, um gegen eine geneigte Oberfläche (20) auf der ersten Stützplatte (12) zu gleiten;
wobei das Scharnier (16) in einer länglichen Öffnung (24) gelagert ist, die in einem Scharniergehäuse (26) ausgebildet ist, das aus der ersten Stützplatte (12) herausragt, wobei das Scharnier (16) frei ist, sich in der länglichen Öffnung (24) zu verschieben und zu drehen,
**dadurch gekennzeichnet, dass** das Wirbelsäulen-Cage (10) ein oder mehrere Stützelemente (28) umfasst, die in Richtung des Scharniers (16) und/oder der ersten Stützplatte (12) vorgespannt sind.

2. Wirbelsäulen-Cage (10) nach Anspruch 1, wobei das eine oder die mehreren Stützelemente (28) aus einem flexiblen Material bestehen.

3. Wirbelsäulen-Cage (10) nach Anspruch 1, wobei, wenn das Wirbelsäulen-Cage (10) in einer zusammengezogenen Konfiguration vorliegt, das eine oder die mehreren Stützelemente (28) an dem Scharnier (16) und/oder der ersten Stützplatte (12) anstoßen.

4. Wirbelsäulen-Cage (10) nach Anspruch 1, wobei sich das eine oder die mehreren Stützelemente (28) in einer Verschiebung und/oder einer Drehung bewegen.

5. Wirbelsäulen-Cage (10) nach Anspruch 1, wobei, wenn die erste Stützplatte (12) in Bezug auf die zweite Stützplatte (14) angehoben und/oder gekippt wird, ein Spalt zwischen dem Scharnier (16) und der zweiten Stützplatte (14) und/oder zwischen der ersten Stützplatte (12) und der zweiten Stützplatte (14) ausgebildet wird und die einen oder die mehreren Stützelemente (28) durch ein Vorspannen gedrängt werden, sich in den Spalt zu bewegen.

6. Wirbelsäulen-Cage (10) nach Anspruch 1 oder 5, wobei das eine oder die mehreren Stützelemente (28) durch eine Vorspannvorrichtung (30) vorgespannt sind.

7. Wirbelsäulen-Cage (10) nach Anspruch 6, wobei die Vorspannvorrichtung (30) eine Feder umfasst.

8. Wirbelsäulen-Cage (10) nach Anspruch 1 oder 5, wobei die geneigte Oberfläche (20) unterschiedliche Steigungen an unterschiedlichen Abschnitten davon aufweist.

9. Wirbelsäulen-Cage (10) nach Anspruch 1 oder 5, wobei der Plattenbeweger (18) unterschiedliche Steigungen an unterschiedlichen Abschnitten davon aufweist.

10. Wirbelsäulen-Cage (10) nach Anspruch 1 oder 5, das ferner einen oder mehrere Kiele (32) umfasst, die sich von der ersten Stützplatte (12) und/oder der zweiten Stützplatte (14) erstrecken.

11. Wirbelsäulen-Cage (10) nach Anspruch 1 oder 5, das ferner innere Rampen (40) umfasst, auf denen sich die erste Stützplatte (12) bewegt.

12. Wirbelsäulen-Cage (10) nach Anspruch 1 oder 5, das ferner ein Applikator (42) mit einem proximalen Trichter (43) und einem Einstellinstrument (44) zum Durchleiten des Applikator (42) umfasst.

13. Wirbelsäulen-Cage (10) nach Anspruch 5, wobei das eine oder die mehreren Stützelemente (28) aus einem flexiblen Material bestehen.

14. Wirbelsäulen-Cage (10) nach Anspruch 5, wobei, wenn der Wirbelsäulen-Cage (10) in einer zusammengezogenen Konfiguration vorliegt, das eine oder die mehreren Stützelemente (28) an dem Scharnier (16) und/oder der ersten Stützplatte (12) anstoßen.

15. Wirbelsäulen-Cage (10) nach Anspruch 5, wobei sich das eine oder die mehreren Stützelemente (28) in einer Verschiebung und/oder einer Drehung bewegen.

## Revendications

1. Cage vertébrale (10) comprenant :
une première plaque de support (12) reliée de manière pivotante à une seconde plaque de support (14) par une charnière (16) ; et
un appareil de déplacement de plaque (18) actionné par un actionneur (22) et situé entre lesdites première et seconde plaques de support (12, 14), ledit appareil de déplacement de plaque (18) étant agencé pour coulisser contre une surface inclinée (20) sur ladite première plaque de support (12) ;
ladite charnière (16) étant montée dans une ouverture oblongue (24) formée dans un boîtier de charnière (26) faisant saillie de ladite première plaque de support (12), ladite charnière (16) étant libre de translater et de tourner dans ladite ouverture oblongue (24),
**caractérisée en ce que** ladite cage vertébrale (10) comprend un ou plusieurs éléments de support (28) sollicités vers ladite charnière (16) et/ou ladite première plaque de support (12).

2. Cage vertébrale (10) selon la revendication 1, dans laquelle lesdits un ou plusieurs éléments de support (28) sont constitués d'un matériau flexible.

3. Cage vertébrale (10) selon la revendication 1, dans laquelle, lorsque ladite cage vertébrale (10) est dans une configuration contractée, lesdits un ou plusieurs éléments de support (28) butent contre ladite charnière (16) et/ou ladite première plaque de support (12).

4. Cage vertébrale (10) selon la revendication 1, dans laquelle lesdits un ou plusieurs éléments de support (28) se déplacent en translation et/ou en rotation.

5. Cage vertébrale (10) selon la revendication 1, dans laquelle, lorsque ladite première plaque de support (12) est soulevée et/ou penchée par rapport à ladite seconde plaque de support (14), un espace est formé entre ladite charnière (16) et ladite seconde plaque de support (14), et/ou entre ladite première plaque de support (12) et ladite seconde plaque de support (14), et lesdits un ou plusieurs éléments de support (28) sont poussés par une force de sollicitation à se déplacer dans ledit espace.

6. Cage vertébrale (10) selon la revendication 1 ou la revendication 5, dans laquelle lesdits un ou plusieurs éléments de support (28) sont sollicités par un dispositif de sollicitation (30).

7. Cage vertébrale (10) selon la revendication 6, dans laquelle ledit dispositif de sollicitation (30) comprend un ressort.

8. Cage vertébrale (10) selon la revendication 1 ou la revendication 5, dans laquelle ladite surface inclinée (20) a des plans différents à différentes parties de celle-ci.

9. Cage vertébrale (10) selon la revendication 1 ou la revendication 5, dans laquelle ledit appareil de déplacement de plaque (18) a des plans différents à différentes parties de celui-ci.

10. Cage vertébrale (10) selon la revendication 1 ou la revendication 5, comprenant en outre une ou plusieurs quilles (32) s'étendant depuis ladite première plaque de support (12) et/ou ladite seconde plaque de support (14).

11. Cage vertébrale (10) selon la revendication 1 ou la revendication 5, comprenant en outre des rampes intérieures (40) sur lesquelles se déplace ladite première plaque de support (12).

12. Cage vertébrale (10) selon la revendication 1 ou la revendication 5, comprenant en outre un outil applicateur (42) doté d'un entonnoir proximal (43) et un outil de réglage (44) pour passer à travers ledit outil applicateur (42).

13. Cage vertébrale (10) selon la revendication 5, dans laquelle lesdits un ou plusieurs éléments de support (28) sont constitués d'un matériau flexible.

14. Cage vertébrale (10) selon la revendication 5, dans laquelle, lorsque ladite cage vertébrale (10) est dans une configuration contractée, lesdits un ou plusieurs éléments de support (28) butent contre ladite charnière (16) et/ou ladite première plaque de support (12).

15. Cage vertébrale (10) selon la revendication 5, dans laquelle lesdits un ou plusieurs éléments de support (28) se déplacent en translation et/ou en rotation.
